# EUROPEAN PATENT APPLICATION

(11) **EP 3 494 888 A1**
(43) Date of publication of application: **12.06.2019**
(21) Application number: 17205628.5
(22) Date of filing: 06.12.2017
(51) Int. Cl.: A61B 6/02, A61B 6/04, A61B 6/00, A61B 5/00, G06T 5/50, G06T 11/00, A61B 90/10, G06T 7/30

(54) **METHOD FOR CREATING A SCAN OF AN INTRACEREBRAL STRUCTURE**

(71) Applicant: Medizinische Universität Wien, 1090 Wien (AT)
(72) Inventor: Alesch, François, 3413 Kirchbach (AT)
(74) Representative: Weiser & Voith Patentanwälte Partnerschaft

(57) **Abstract**

The invention relates to a method for creating a scan (S) of an intracerebral structure (T) of a proband's head (3), comprising:
from an emitter (1), emitting electromagnetic radiation (2) in a scanning direction (D) through the proband's head (3) onto an imaging device (7),
in the imaging device (7), generating a first image (IMG₁) by recording the electromagnetic radiation (2) in an image plane (PL) that is non-parallel to said scanning direction (D), wherein the proband's head (3) is depicted at a first position (p₁) in the first image (IMG₁),
inducing a relative movement (8, 9, 12, 14) between the imaging device (7) and the proband's head (3),
generating a second image (IMG₂), in which the proband's head (3) is depicted at a different second position (p₂), and
creating the scan (S) by congruently superposing the first and the second image (IMG₁, IMG₂).

## Description

The present invention relates to a method for creating a scan of an intracerebral structure of a proband's head.

Such scans of a head depicting the intracerebral structure are used when performing stereotactic procedures, for example. An exact knowledge of the intracerebral structure is necessary to locate and reference intracerebral landmarks of the proband's brain.

Stereotactic procedures are usually done using a stereotactic frame fixated at the proband's head. The stereotactic frame serves multiple purposes: it fixates the proband's head, allows a stable mounting to the operation table, serves as an instrument holder during the procedure, and allows a referencing of the proband's intracerebral structures that are the base for target calculation of the stereotactic procedure. The referencing is done by special localisers that serve as an interface to imaging, for example Magnetic Resonance Imaging (MRI), Computer Tomography (CT), or fluoroscopy.

In fluoroscopy, the contrast of the intracerebral structures is particularly low such that the contrast has to be enhanced, which is done, according to the state of the art, by injecting contrast medium into the cerebral ventricles. This method, called ventriculography, is a standard procedure that has several drawbacks: it is an invasive procedure and exposure to contrast medium bares a risk for the proband.

It is an object of the invention to create a method to enhance the contrast of a scan of an intracerebral structure without the need for invasive procedures such as the injection of a contrast medium.

In a first aspect, this object is achieved by a method of the type mentioned at the outset, comprising:
from an emitter, emitting electromagnetic radiation in a scanning direction through the proband's head onto an imaging device,
in the imaging device, generating a first image by recording the electromagnetic radiation in an image plane that is non-parallel to said scanning direction, wherein the proband's head is depicted at a first position in the first image,
characterized by
inducing a relative movement between the imaging device and the proband's head,
generating a second image, in which the proband's head is depicted at a different second position, and
creating the scan by congruently superposing the first and the second image.

According to this method, the contrast of the scan of an intracerebral structure is enhanced by a superposition of images in which the proband's head is depicted at different positions. The invention thus achieves a non-invasive method for creating a scan of higher contrast such that the intracerebral structure has a better visibility in the scan while the remainder of the brain is less visible.

The invention provides various embodiments to achieve said different positions of the proband's head in the images. The embodiments can be split up in two categories as follows.

In the first category of embodiments, for inducing the relative movement, the proband's head is moved and the second image is generated by recording the electromagnetic radiation in the image plane such that the proband's head is depicted at said second position. The movement of the proband's head, which can be applied as a light vibration by micro-shifting, for example, prevents from moving (or vibrating) equipment with technically sensitive components.

Preferably, the proband's head is fixated by a stereotactic frame, which is displaced to move the proband's head. This facilitates the accurate control of the movement onto the proband's head as necessary for creating the scan. Thus, the stereotactic frame serves two purposes, namely on the one hand as a reference frame for the placement of the instruments and secondly as a movement-inducing tool.

In the second category of embodiments, said different positions are achieved by applying movement to the imaging device. Thereby, no movement has to be applied to the proband's head. Also in this case, the movement can be applied as a light vibration by micro-shifting. For the movement of the imaging device, the following variants are preferred.

In the first variant, for inducing the relative movement, the imaging device itself is moved and the second image is generated by recording the electromagnetic radiation in the image plane such that the proband's head is depicted at said second position. This has the advantage that a standard imaging device can be used, which does not have to be specifically adapted to the inventive method.

In the second variant, for inducing the relative movement, an optic of the imaging device is moved and the second image is generated by recording the electromagnetic radiation in the image plane such that the proband's head is depicted at said second position. By moving the optic, the total mass of moved elements and consequently the installed power is reduced and thus energy and space can be conserved. The optic can be moved in a simple manner, e.g., by a tilting motor. All electronic components can be kept statically, which improves the lifespan of the imaging device.

In the third variant, for inducing the relative movement, a detection array within the imaging device is moved and the second image is generated by recording the electromagnetic radiation in the image plane such that the proband's head is depicted at said second position. This variant has the advantage that only the detection array has to be moved, e.g., with a lateral displacement motor, while all other components of the imaging device can be kept still, which is more efficient than moving the whole imaging device and thus conserves energy and space. Also, the movement of small components such as the detection array and/or the optic is easier controllable, yielding a more accurate control and an improved scan.

In the above variants, the relative movement is preferably induced by a vibration. Thereby, the movement can be kept within a predefined range, e.g., with predefined amplitudes. Also, a centre of the vibration can be defined as a reference zero position such that the proband's head or the imaging device can be moved back to this position after creating the scan. The intracerebral structure can then be referenced back to the proband's head by means of the stereotactic frame.

The vibration is preferably a linear vibration, which makes it easier to control and predetermine in which positions the head will be in the images. Alternatively, non-linear vibrations can be utilised such as elliptically rotating vibrations, which allows for more degrees of freedom and is especially favourable when more than two images are recorded for generating the scan.

Furthermore, in a preferred embodiment, the first position and the second position are spaced apart from one another by a distance corresponding to a predetermined parameter of the intracerebral structure. By inducing a relative movement based on said parameter, the contrast of the scan can be enhanced. If on the other hand said distance is too high or too low, the contrast of the scan will be lower, too. The predetermined parameter can, e.g., be a thickness of the intracerebral structure of the proband's head. A determination of the magnitude of the relative movement to be induced to achieve this enhancement is straight-forward.

Favourably, the images each have a resolution of 1024 x 1024 pixels. This allows to accurately visualize the intracerebral structure while keeping the hardware requirements low.

In this embodiment, it is preferred that said first and said second position are spaced apart from each other by 2 to 20 pixels. In general, it is preferred that the first and the second position are spaced apart by 0,2 - 2 % of the image size. This is achieved by micro-shifting the proband's head or the imaging device (or its components), respectively. Scans created this way have shown to have especially suitable contrast values.

Preferably, the method is used to superpose not only two but a plurality of generated images. To this end, in total at least ten images, preferably at least fifty images, are generated, wherein the proband's head is depicted at a different position in each image, and all of said generated images are superposed to create the scan. With the number of pictures, the contrast of the scan and thus the visibility of the intracerebral structure increases, too.

In a further aspect, the invention relates to a method for obtaining a depth-scan of an intracerebral structure of a proband's head, wherein the method according to any one of the aforementioned embodiments is performed twice, once while said scanning direction is a first direction through the proband's head for creating a first scan and once while the scanning direction is a different second direction through the proband's head for creating a second scan, wherein the first and the second scan are combined to obtain the depth-scan.

This has the advantage that two scans with improved contrast taken from different angles can be combined to extract depth information. The depth information can then in turn be used to establish a three dimensional model of the intracerebral structure of the proband's head. The depth information as well as the three dimensional model allow for a better determination of the intracerebral structure and thus more accurate guidance for operations.

Advantageously, the first direction is the anteroposterior direction of the proband's head and the second direction is the lateral direction of the proband's head. This facilitates firstly the positioning of the proband as directions are used that are predetermined by the proband's anatomy and secondly the evaluation of the scans. Because the scans are taken from orthogonal directions, the combination of the scans allows for determining the depth information accurately and without much computational effort.

In order to change the scanning direction between said first and said second direction, the proband and/or his head can be turned. However, preferably, the emitter and the imaging device are turned 90° around the proband's head between creating the first and the second scan. This is advantageous because the proband does not have to be moved during taking the scans. The emitter and imaging device can for this purpose be mounted rotatably with the rotation axis substantially through the proband's head to allow for a rotation of the emitter and imaging device on the fly for a quick and easy re-positioning. This allows to create the scans in close temporal proximity.

The invention shall now be explained in more detail below on the basis of preferred exemplary embodiments thereof with reference to the accompanying drawings, in which:
Fig. 1 shows a system for creating a scan of an intracerebral structure according to the present invention in a schematic side view;
Fig. 2 shows a superposing of two images according to the invention in a sequence of schematic images;
Fig. 3 shows details of the superposing of two images according to Fig. 2; and
Fig. 4 shows a system for creating a depth scan of an intracerebral structure according to the present invention in a schematic top view.

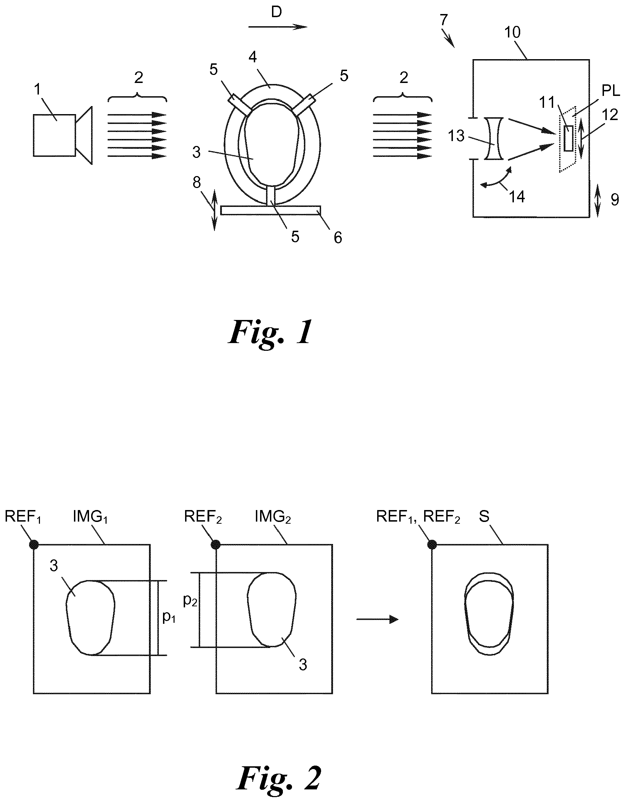

Fig. 1 shows an emitter 1 that emits electromagnetic radiation 2, e.g., X-rays, in a scanning direction D, for creating a scan S of an intracerebral structure T of a proband's head 3, which is located in the scanning direction D as seen from the emitter 1. During the scan S, the proband's head 3 is optionally fixated in a stereotactic frame 4 with multiple fixating elements 5 and a base member 6 as known in the state of the art. The stereotactic frame 4 can be used as a referencing tool for performing operations after the scan S has been created, when necessary.

After the electromagnetic radiation 2 has passed the proband's head 3, it is recorded by means of an imaging device 7, which can be a common X-ray detector, for example. The imaging device 7 records the electromagnetic radiation 2 in an image plane PL, which is non-parallel to the scanning direction D, in typical embodiments substantially orthogonal thereto. Thereby, a first image IMG₁, also known as "radiograph" in radiology, is generated by the imaging device 7.

Fig. 2 shows on the left the first image IMG₁ generated by the imaging device 7. In the first image IMG₁ the proband's head 3 is depicted at a first position p₁. The intracerebral structure T in the proband's head 3 is barely visible in the first image IMG₁ and is hard to detect even for skilled physicians.

To enhance the contrast of the intracerebral structure T in the scan S, a relative movement is induced between the imaging device 7 and the proband's head 3, and a second image IMG₂ is generated, in which the proband's head 3 is depicted at a second position p₂ that differs from the first position p₁. To create a scan S, the first image IMG₁ and the second image IMG₂ are congruently superposed such that the difference of positions p₁, p₂ remains.

The positions p₁, p₂ can, e.g., be referenced from respective reference points REF₁, REF₂ of the image IMG₁, IMG₂; in the example of Fig. 2 the reference points REF₁, REF₂ are the left upper corner. When superposing the images IMG₁, IMG₂ congruently to create the scan S, the reference points REF₁, REF₂ of the two images IMG₁, IMG₂ coincide.

For obtaining the different positions p₁, p₂ in the images IMG₁, IMG₂, various embodiments that fall in two categories are possible and described in the following. The first category comprises a movement of the proband's head 3 while the second category comprises a movement of the imaging device 7.

Coming back to Fig. 1, the difference in positions p₁, p₂ in the images IMG₁, IMG₂ is achieved, according to said first category, by means of moving the proband's head 3 for inducing the relative movement. When the head 3 has been moved, the second image IMG₂ is generated by recording the electromagnetic radiation 2 in the image plane PL analogously to generating the first image IMG₁.

For moving the head 3, in the example of Fig. 1 the base 6 of the stereotactic frame 4 is used. The movement of the base 6 of the stereotactic frame 4, which in turn causes a movement of the head 3 fixated thereon, is indicated by a double-arrow 8. Alternatively, the proband's head 3 can be moved inside the frame 4 or without using the frame 4.

Regardless of whether the frame 4 or the head 3 directly is to be moved, the movement can be induced by vibrating the frame 4 or the head 3, respectively, by means of a vibrating type motor as known in the state of the art. The vibration can be a linear vibration, e.g., linear in a direction substantially normal to the scanning direction D. Furthermore, the vibration could also be within a plane that is, e.g., normal to the scanning direction D such as a circular or elliptical rotation. Also, a three dimensional vibration of the proband's head 3 around a predetermined point could be utilized.

The magnitude of the relative movement can be chosen such that the first and the second position p₁, p₂ are spaced apart from one another by a distance corresponding to a predetermined parameter of the intracerebral structure, e.g., the thickness of the intracerebral structure. In an exemplary case, the first and second position p₁, p₂ may correspond to the respective maximum amplitudes of said linear vibration. The relation between the positions p₁, p₂ and the relative movement further depends on the propagation and possible deflection of the radiation 2 between the proband's head 3 and the imaging device 7, e.g., caused by a lens between the proband's head and the imaging device 3 or by a divergence of the electromagnetic radiation 2 as emitted by the emitter 1.

Fig. 1 also depicts said second category of embodiments, wherein the different positions p₁, p₂ in the images IMG₁, IMG₂ is achieved by moving the imaging device 7 or components thereof for inducing said relative movement. Firstly, the imaging device 7 itself can be moved, indicated by double-arrow 9 in Fig. 1. This can be done, for example, by applying a vibration onto a housing 10 of the imaging device 7.

Alternatively, components inside the housing 10 of the imaging device 7 can be moved to create different positions p₁, p₂ of the proband's head 3 in the images IMG₁, IMG₂. For example, a detection array 11 can be moved transversally to the scanning direction D, e.g., in a translation motion, indicated by a double-arrow 12 in Fig. 1. Alternatively, an optic 13 focussing the electromagnetic radiation 2 onto the detection array 11 can be moved, indicated by a double-arrow 14. If the optic 13 is to be moved, tilting can optionally be preferred over translation.

As explained above, the imaging device 7 can be of any kind known in the art and as such the detection array 11 can be an imaging sensor that converts the electromagnetic radiation 2 into electric impulses for electronic processing or it can simply be a photographic plate or photographic film. The optic 13, on the other hand, can be a focussing lens, mirror system, or the like.

For the movements indicated by the double-arrows 9, 12, 14, again vibrational movements can optionally be utilized.

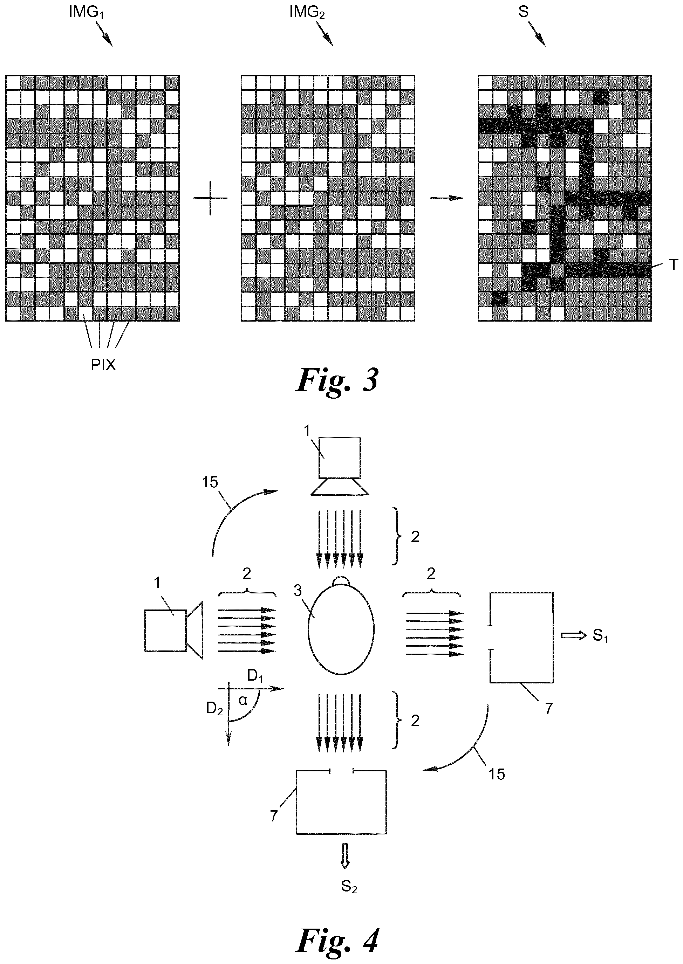

Fig. 3 shows an overlay of the first image IMG₁ and the second image IMG₂ to generate the scan S in more detail. The second image IMG₂ was generated after the relative movement had been induced by moving the proband's head 3 as described above. Here, both the first image IMG₁ and the second image IMG₂ comprise a predefined number of pixels PIX, e.g., 1024 x 1024.

To create the scan S with enhanced contrast of the intracerebral structure T, the images IMG₁, IMG₂ are superposed congruently as in the embodiments of other categories. Here, but also in all other embodiments, superposed means any combination of the images IMG₁, IMG₂ such that the features of both images IMG₁, IMG₂ appear in the created scan S. For example, the pixel values of the scan S can be the average of a pixel value of the first image IMG₁ and the corresponding pixel value of the second image IMG₂. Alternatively, instead of an average also a pure addition of pixel values can be performed such as shown in Fig. 3, where grey pixel values that occur in the first image IMG₁ as well as in the second image IMG₂ become black in the superposition of the created scan S.

In the example of Fig. 3, the difference of the first and the second position p₁, p₂ is one pixel. Depending on the size of the images IMG₁, IMG₂, the difference can vary and be in the range of 0,2 - 2 % of the size of the image, for example. To achieve this, the proband's head 3 or the imaging device 7 is subject to micro-shifting. If the images IMG₁, IMG₂ have a resolution of, e.g., 1024 x 1024 pixels, the first and second positions p₁, p₂ are spaced apart from each other, i.e., yielding a pixel shift, by 2 to 20 pixels.

It is to be understood that not only two but multiple images IMG₁, IMG₂, ..., generally IMGᵢ, e.g., at least ten or fifty images IMGᵢ, can be generated according to any of the above embodiments. In all of those multiple images IMGᵢ, the proband's head 3 is then depicted at a different position p₁, p₂, ..., generally pᵢ, such that the contrast of the scan S and the visibility of the intracerebral structure T is even higher than with two images IMG₁, IMG₂ when superposing all of said generated images IMGᵢ to create the scan S.

Fig. 4 shows the creation of a depth scan by means of performing the above method twice, once while the scanning direction D is a first direction D₁, i.e., emitting the electromagnetic radiation 2 in the first direction D₁, for creating a first scan S₁ and once while the scanning direction D is a second direction D₂, i.e., emitting the electromagnetic radiation 2 in the second direction D₂, for creating a second scan S₂.

After creating the first and the second scan S₁, S₂, they are combined to create a depth scan of the intracerebral structures T within the proband's head 3. The depth scan is in the simplest case created in that a point, i.e., an intracerebral landmark, is identified in the first scan S₁, for example by assigning it x- and y-coordinates. Thereupon, a depth information is inferred for this point by locating the same intracerebral landmark in the second scan S₂ to assign it a z-coordinate, for example. It can be seen that this is especially easy when the first and the second direction D₁, D₂ are orthogonal, i.e., when they enclose an angle α of 90°.

An even more elaborate depth scan could be the creation of a whole three dimensional model of the intracerebral structure T of the proband's head 3 by applying the coordinate scheme above for multiple intracerebral landmarks.

From Fig. 4 it can further be seen that the first direction D₁ runs from the left to the right, which here corresponds to the lateral direction of the proband's head 3. The result is a lateral scan S₁, which depicts the proband's head 3 and its intracerebral structure T from the side. The second direction D₂ running from top to bottom in Fig. 4, which here corresponds to the anteroposterior direction of the proband's head 3 for creating an anteroposterior scan S₂.

The movement 15 of the emitter 1 and the imaging device 7 can be performed of any kind as known in X-ray radiology. Here the emitter 1 and the imaging device 7 can, e.g., be mounted on a common carrier (not shown), which has a rotation axis running substantially through the proband's head 3. The emitter 1 and the imaging device 7 can then be turned 90° around the proband's head 3 between creating the first and the second scan S₁, S₂.

Alternatively to rotating the emitter 1 and imaging device 7 around the proband's head 3, both components could also be kept still and the proband rotates by 90° on the spot, e.g., by means of a rotating chair or the like. Further alternatively, two sets of emitters 1 and imaging devices 7 could be used to concurrently create the first and second scan S₁, S₂. In this case, neither the emitter 1 and the imaging device 7 nor the proband 3 would have to be moved.

The invention is not restricted to the specific embodiments described in detail herein, but encompasses all variants, combinations and modifications thereof that fall within the framework of the appended claims.

## Claims

1. Method for creating a scan (S) of an intracerebral structure (T) of a proband's head (3), comprising:
from an emitter (1), emitting electromagnetic radiation (2) in a scanning direction (D) through the proband's head (3) onto an imaging device (7),
in the imaging device (7), generating a first image (IMG₁) by recording the electromagnetic radiation (2) in an image plane (PL) that is non-parallel to said scanning direction (D), wherein the proband's head (3) is depicted at a first position (p₁) in the first image (IMG₁),
**characterized by**
inducing a relative movement (8, 9, 12, 14) between the imaging device (7) and the proband's head (3),
generating a second image (IMG₂), in which the proband's head (3) is depicted at a different second position (p₂), and
creating the scan (S) by congruently superposing the first and the second image (IMG₁, IMG₂).

2. Method according to claim 1, wherein, for inducing the relative movement (8, 9, 12, 14), the proband's head (3) is moved (8) and the second image (IMG₂) is generated by recording the electromagnetic radiation (2) in the image plane (PL) such that the proband's head (3) is depicted at said second position (p₂).

3. Method according to claim 2, wherein the proband's head (3) is fixated by a stereotactic frame (4), which is displaced to induce the relative movement (8, 9, 12, 14).

4. Method according to claim 1, wherein, for inducing the relative movement (8, 9, 12, 14), the imaging device (7) itself is moved (9) and the second image (IMG₂) is generated by recording the electromagnetic radiation (2) in the image plane (PL) such that the proband's head (3) is depicted at said second position (p₂).

5. Method according to claim 1, wherein, for inducing the relative movement (8, 9, 12, 14), an optic (13) of the imaging device (7) is moved (13) and the second image (IMG₂) is generated by recording the electromagnetic radiation (2) in the image plane (PL) such that the proband's head (3) is depicted at said second position (p₂).

6. Method according to claim 1, wherein, for inducing the relative movement (8, 9, 12, 14), a detection array (11) within the imaging device (7) is moved (12) and the second image (IMG₂) is generated by recording the electromagnetic radiation (2) in the image plane (PL) such that the proband's head (3) is depicted at said second position (p₂).

7. Method according to any one of the claims 2 - 6, wherein the relative movement (8, 9, 12, 14) is induced by a vibration.

8. Method according to claim 7, wherein the vibration is a linear vibration.

9. Method according to any one of the claims 1 - 8, wherein the first position (p₁) and the second position (p₂) are spaced apart from one another by a distance corresponding to a predetermined parameter of the intracerebral structure.

10. Method according to any one of the claims 1 - 9, wherein the images (IMG₁, IMG₂) each have a resolution of 1024 x 1024 pixels (PIX).

11. Method according to claim 10, wherein said first and said second position (p₁, p₂) are spaced apart from each other by 2 to 20 pixels.

12. Method according to any one of the claims 1 - 11, wherein in total at least ten images, preferably at least fifty images, are generated, wherein the proband's head (3) is depicted at a different position in each image,
and all of said generated images are superposed to create the scan (S).

13. Method for obtaining a depth-scan of an intracerebral structure (T) of a proband's head (3),
wherein the method according to any one of the claims 1 - 12 is performed twice, once while said scanning direction (D) is a first direction (D₁) through the proband's head (3) for creating a first scan (S₁) and once while the scanning direction (D) is a different second direction (D₂) through the proband's head (3) for creating a second scan (S₂),
wherein the first and the second scan (S₁, S₂) are combined to obtain the depth-scan.

14. Method according to claim 13, wherein the first direction (D₁) is the anteroposterior direction of the proband's head (3) and the second direction (D₂) is the lateral direction of the proband's head (3).

15. Method according to claim 13 or 14, wherein the emitter (1) and the imaging device (7) are turned 90° around the proband's head (3) between creating the first and the second scan (S₁, S₂).
